# EUROPEAN PATENT SPECIFICATION

(11) **EP 2 532 298 B1**
(45) Date of publication and mention of the grant of the patent: **13.11.2013**
(21) Application number: 12170062.9
(22) Date of filing: 30.05.2012
(51) Int. Cl.: A61B 1/00, A61B 1/12

(54) **Fluid nozzle unit and endoscope**
Flüssigkeitsdüsenvorrichtung und Endoskop
Unité de buse de fluide et endoscope

(30) Priority: 08.06.2011 JP 2011128334
(43) Date of publication of application: 12.12.2012
(73) Proprietor: Fujifilm Corporation, Minato-ku Tokyo (JP)
(72) Inventor: Koga, Takehiko, Kanagawa (JP)
(74) Representative: Klunker . Schmitt-Nilson . Hirsch

(56) References cited:
- JP-A- 9 075 297
- JP-U- 57 042 803
- US-A1- 2009 253 965

## Description

### BACKGROUND OF THE INVENTION

### 1. Field of the Invention

The present invention relates to a fluid nozzle unit and an endoscope according to the preamble of claim 1. More particularly, the present invention relates to a fluid nozzle unit which is used with an endoscope and can be cleaned easily, and an endoscope having the fluid nozzle unit.

### 2. Description Related to the Prior Art

An endoscope includes an elongated tube or guide tube, an imaging window, lighting windows and a fluid nozzle. The elongated tube is entered in a body cavity of a patient. The imaging window, the lighting windows and the fluid nozzle are disposed at a distal end of the elongated tube. The imaging window receives object light from an object in the body cavity. The lighting windows apply light to the body cavity. The fluid nozzle ejects fluid to the imaging window, for example, washing water and air. A surface of the imaging window appears externally at the distal end. Dirt or body fluid from the body cavity is likely to deposit on the surface of the imaging window. Thus, a spout of the fluid nozzle ejects fluid to remove the dirt from the imaging window.

JP-A 2005-000567 (corresponding to JP-B 4332710 and DE-A 10 2004 029 099) and JP-A 8-140926 disclose an endoscope in which an end shell is disposed at a distal end of the elongated tube, and the fluid nozzle is removably secured to the end shell. If the dirt or body fluid from the body cavity is stuck inside the fluid nozzle, the fluid nozzle is exchanged. In the end shell are formed flow lines for supply of water and air and a converging channel in a Y shape for the flow lines. When the fluid nozzle is mounted on the end shell, the fluid nozzle communicates with the converging channel. The water and air from the flow lines flow through the converging channel and are ejected by the fluid nozzle.

In JP-A 2005-000567 and JP-A 8-140926, however, there is no solution of a problem of deep entry of dirt into the converging channel or the flow lines after passage through the fluid nozzle. An open area of the converging channel in the end shell is so small that a brush or other cleaning tool cannot reach the converging channel easily. Operation of cleaning the converging channel is extremely difficult. Also, it is difficult for an operator visually to check the dirt or body fluid inside the flow lines which is disposed behind the converging channel. Cleaning the flow lines is very hard.

In accordance with the preamble of claim 1, US 2009/0253965 A1 discloses a fluid nozzle unit. In this prior art unit, there is provided a jet outlet which directs a jet of fluid and/or gas towards the imaging window means.

JP 9 075 297 A discloses a nozzle unit having a spout received by a distal end of an elongated tube of an endoscope.

JP 357 042 803 U discloses a nozzle unit having two conduits.

### SUMMARY OF THE INVENTION

In view of the foregoing problems, an object of the present invention is to provide a fluid nozzle unit which is used with an endoscope and can be cleaned easily, and an endoscope having the fluid nozzle unit.

In order to achieve the above and other objects and advantages of this invention, a fluid nozzle unit for an endoscope has the features defined by claim 1.

The endoscope includes a holder cavity formed in the end shell to open in the distal surface. The nozzle housing is contained in the holder cavity.

Furthermore, a retaining hole is formed in the nozzle housing to communicate from the converging channel toward the distal surface. The nozzle spout device includes a port sleeve, retained in the retaining hole, and connected with the converging channel. A spout sleeve is disposed on the distal surface to extend from a distal end of the port sleeve toward the imaging window area.

Furthermore, a positioning device positions the nozzle housing in the holder cavity to prevent drop therefrom.

Furthermore, a sealing device is disposed on a peripheral surface of the nozzle housing, for hermetically closing a gap between the nozzle housing and an inner surface of the holder cavity.

Furthermore, a peripheral groove is formed in the peripheral surface, for receiving the sealing device. The endoscope includes an internal groove, formed in the inner surface of the holder cavity, opposed to the peripheral groove, for receiving the sealing device so as to position the nozzle housing in the holder cavity.

The check valve device includes a valve opening for passing the liquid from the liquid channel. A valve seat is disposed at the valve opening. A valve membrane is secured to the valve opening, shiftable from a closed position to an open position upon being pressed, for engagement with the valve seat when in the closed position, to close the valve opening and prevent passage of liquid from the converging channel, and for coming away from the valve seat when in the open position, to open the valve opening.

The endoscope includes a head cap, secured to the end shell, and positioned on the distal surface. A first end opening is formed in the head cap, for setting the imaging window area to appear externally. A second end opening is formed in the head cap to correspond to the holder cavity.

Also, an endoscope is provided, and includes the fluid nozzle unit.

Furthermore, a holder cavity is formed in the end shell to open in the distal surface, for containing the nozzle housing.

Furthermore, a positioning device positions the nozzle housing in the holder cavity to prevent drop therefrom.

The positioning device includes a peripheral groove formed in a peripheral surface of the nozzle housing. A positioning projection is formed to project from an inner surface of the holder cavity, and received in the peripheral groove.

Consequently, the flow lines of fluid in the endoscope can be cleaned up even with a simple structure of the fluid nozzle, because of the nozzle housing removably mounted on the endoscope and having the branch conduits.

### BRIEF DESCRIPTION OF THE DRAWINGS

The above objects and advantages of the present invention will become more apparent from the following detailed description when read in connection with the accompanying drawings, in which:
Fig. 1 is a side elevation illustrating an endoscope system;
Fig. 2 is a perspective view illustrating a head assembly of the endoscope;
Fig. 3 is a perspective view illustrating the head assembly and a fluid nozzle unit removed from the head assembly;
Fig. 4 is a vertical section illustrating an end shell, an imaging window area and the fluid nozzle unit;
Fig. 5 is a vertical section illustrating the fluid nozzle unit;
Fig. 6 is a vertical section illustrating another preferred embodiment in which an annular sealing device operates for positioning.

### DETAILED DESCRIPTION OF THE PREFERRED EMBODIMENT(S) OF THE PRESENT INVENTION

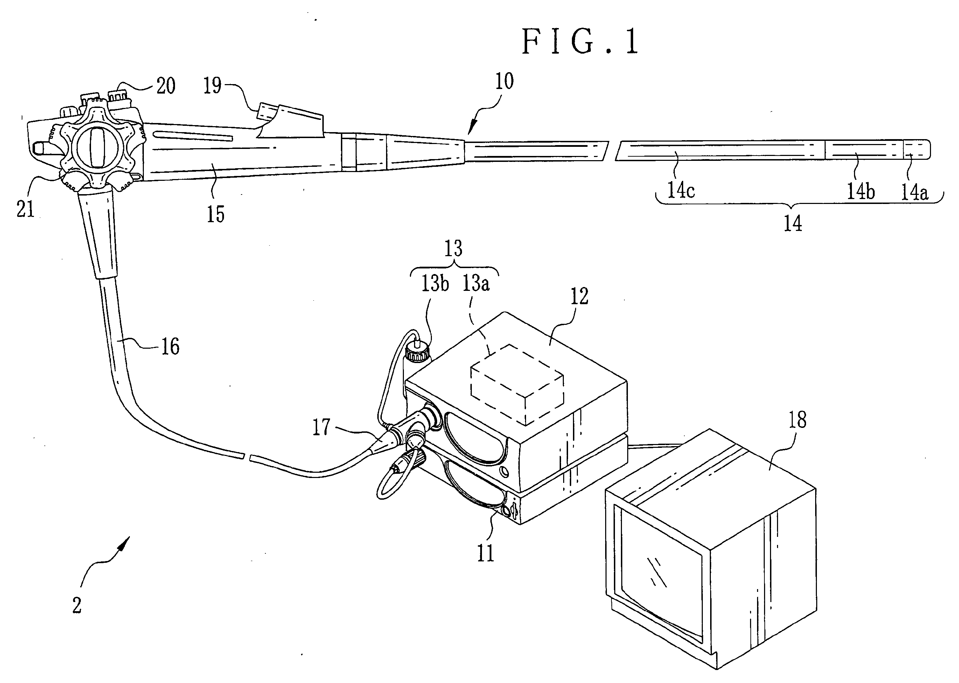

In Fig. 1, an endoscope system 2 includes an electronic endoscope 10, a processing apparatus 11, a light source apparatus 12 and a fluid supply source 13. The fluid supply source 13 includes an insufflator 13a or air pump, and a water tank or reservoir 13b. The insufflator 13a is incorporated in the light source apparatus 12, and supplies air. The water tank 13b is disposed outside the light source apparatus 12, and stores water for cleaning. The endoscope 10 includes a section of an elongated tube 14 or guide tube, a handle 15 and a universal cable 16. The elongated tube 14 is flexible and entered in a body cavity of a patient's body. The handle 15 is disposed at a proximal end of the elongated tube 14. The universal cable 16 is used for connection to the processing apparatus 11 and the light source apparatus 12.

The elongated tube 14 includes a head assembly 14a, a steering device 14b and a flexible device 14c. A CCD image sensor 32 is incorporated in the head assembly 14a for imaging an object. The steering device 14b is disposed at a proximal end of the head assembly 14a. The flexible device 14c is disposed at a proximal end of the steering device 14b.

A connection plug 17 is disposed at a tip of the universal cable 16. The connection plug 17 is in a composite form for connection to the processing apparatus 11, the light source apparatus 12 and the fluid supply source 13.

The processing apparatus 11 receives an image signal from the CCD image sensor 32 through the universal cable 16 and the connection plug 17, and processes the image signal for image processing of various functions, to create image data. The processing apparatus 11 sends a control signal to the CCD image sensor 32. A monitor display panel 18 is connected with the processing apparatus 11 on line, and driven to display an object image according to the image data from the processing apparatus 11 . Also, the processing apparatus 11 is connected with the light source apparatus 12, and controls all the components in the endoscope system 2.

The handle 15 includes a proximal instrument opening 19, fluid supply buttons 20 and steering wheels 21. When the steering wheels 21 are rotated, the steering device 14b is steered up and down and to the right and left, as its wires are pulled or released within the elongated tube 14. Thus, the head assembly 14a is directed in a desired direction in a body cavity.

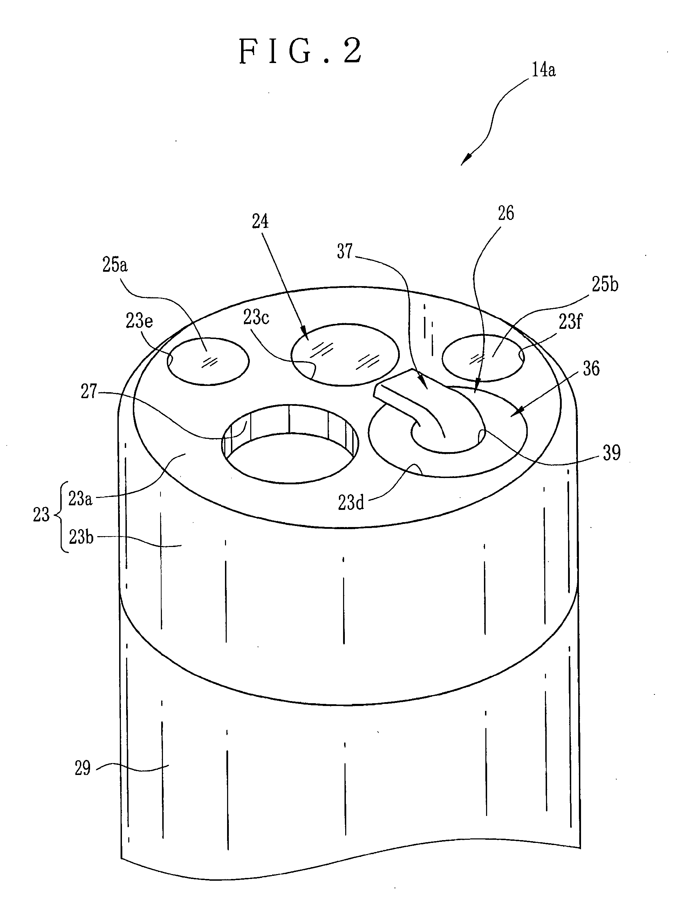

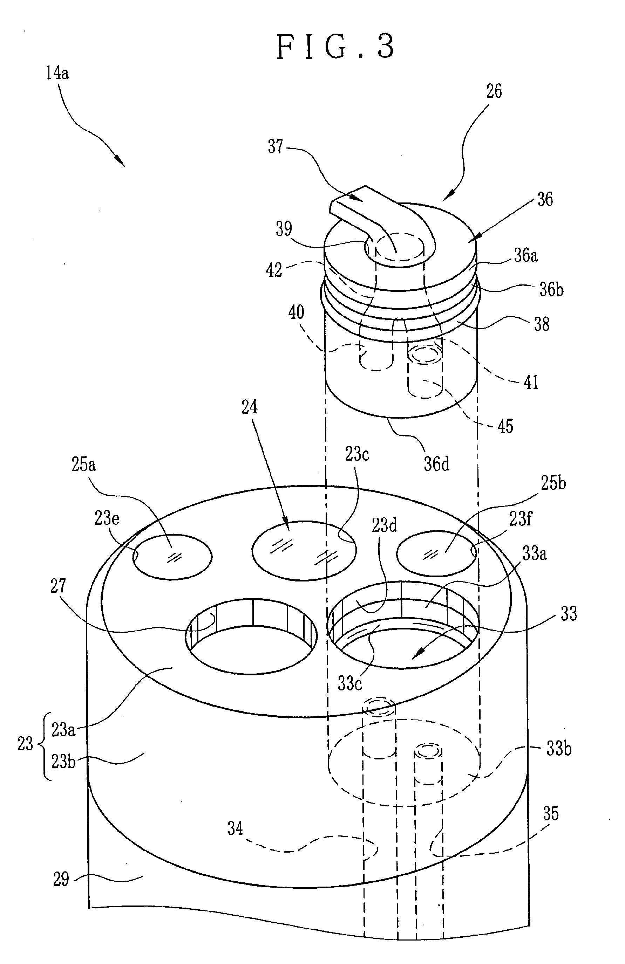

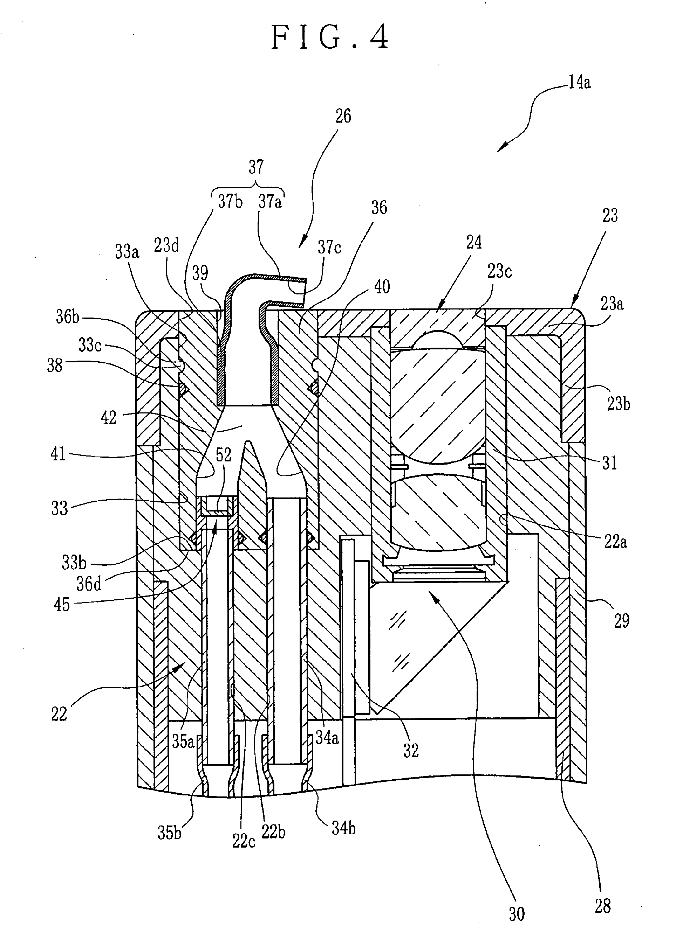

In Figs. 2, 3 and 4, the head assembly 14a includes an end shell 22, a head cap 23, an imaging window area 24, lighting window areas 25a and 25b, a fluid nozzle unit 26 of a cartridge type, and a distal instrument opening 27. The head cap 23 is fitted on the end shell 22. There are plural link elements 28 in the steering device 14b. A proximal end of the end shell 22 is connected with one of the link elements 28 positioned on a distal side.

The head cap 23 includes a cap plate 23a and a cap skirt 23b. The cap plate 23a covers a distal end of the end shell 22. The cap skirt 23b is fitted on a peripheral surface of the end shell 22. A cover tube 29 covers a peripheral surface of the steering device 14b and extends to the end shell 22. A distal end of the cover tube 29 abuts on a proximal end of the cap skirt 23b, and is attached thereto by use of adhesive agent or the like.

The cap plate 23a with the distal instrument opening 27 also includes end openings 23c, 23d, 23e and 23f. The end opening 23c is disposed at a center of the head assembly 14a, and defines the imaging window area 24 on the distal side. The end opening 23d is formed to position the fluid nozzle unit 26. The end openings 23e and 23f are formed to define respectively the lighting window areas 25a and 25b.

An objective lens system 30 is incorporated in the head assembly 14a. A lens/lens group included in the lens system 30 is disposed at a distal end, is mounted in the imaging window area 24, and operates also as a glass cover. A shape of the imaging window area 24 is circular. A lens barrel 31 contains the lens system 30 including the imaging window area 24.

The lens barrel 31 supports a peripheral edge of the lens/lens group of the imaging window area 24. The lens/lens group of the imaging window area 24 is fitted in the end opening 23c of the head cap 23. A holder channel 22a is formed in the end shell 22. The lens barrel 31 is fitted in the holder channel 22a. A distal surface of the lens barrel 31 is contacted tightly by the cap plate 23a of the head cap 23.

Note that the imaging window area 24 may be a flat cover of glass at a distal end of the lens system 30 without function of a lens. Such a flat cover as the imaging window area 24 may not constitute a portion of the lens system 30, and can be directly fitted in the end opening 23c of the head cap 23 for attachment.

The CCD image sensor 32 is disposed downstream of the lens system 30. An example of the CCD image sensor 32 is an interline transfer type. In place of the CCD image sensor 32, a CMOS image sensor can be used.

The lighting window areas 25a and 25b are arranged symmetrically around the center of the imaging window area 24. Light guide devices (not shown) are disposed behind the lighting window areas 25a and 25b. Each of the light guide devices is a bundle of a large number of optical fibers, and passes through the elongated tube 14, the handle 15, the universal cable 16 and the connection plug 17. Light from the light source apparatus 12 is guided by the light guide devices to the lighting window areas 25a and 25b for lighting of an object in a body cavity. Each of the lighting window areas 25a and 25b is constituted by a lens, and applies light from the light source apparatus 12 to the object.

An instrument channel (not shown) is formed through the elongated tube 14. The distal instrument opening 27 is a distal end of the instrument channel. The proximal instrument opening 19 of the handle 15 is its proximal end. A medical instrument, when entered through the proximal instrument opening 19, protrudes from the distal instrument opening 27.

A holder cavity 33 is formed in the end shell 22, and receives entry of the fluid nozzle unit 26 in a removable manner. The fluid nozzle unit 26 is a disposable part. If mucus, body fluid or other dirt deposits on the fluid nozzle unit 26, the fluid nozzle unit 26 is removed from the end shell 22 and abandoned. A new fluid nozzle unit 26 is fitted in the end shell 22.

The holder cavity 33 is formed cylindrically to open in the distal surface of the end shell 22 in an axial direction of the elongated tube 14. A position and diameter of the end opening 23d of the head cap 23 are determined in compliance with the holder cavity 33.

Receiving holes 22b and 22c are formed in the end shell 22 and positioned on a proximal side of the holder cavity 33. An air channel 34 is positioned in the receiving hole 22b. A water channel 35 is positioned in the receiving hole 22c. The air channel 34 is constituted by an air flow sleeve 34a and an air supply tube 34b. The air flow sleeve 34a is a pipe of rigid metal, and fitted in the receiving hole 22b. The air supply tube 34b is coupled with an end of the air flow sleeve 34a, and formed from flexible resin. The water channel 35 is constituted by a water flow sleeve 35a and a water supply tube 35b. The water flow sleeve 35a is a pipe of rigid metal, and fitted in the receiving hole 22c. The water supply tube 35b is coupled with an end of the water flow sleeve 35a, and formed from flexible resin. The supply tubes 34b and 35b are disposed to extend through the elongated tube 14, the handle 15 and the connection plug 17, and supplied with air and water by the fluid supply source 13. Tips of the air flow sleeve 34a and the water flow sleeve 35a protrude through a base surface 33b into the holder cavity 33, and are kept fixed in the end shell 22.

The holder cavity 33 has an inner surface 33a. A snap-fit projection 33c projects annularly from the inner surface 33a. A nozzle housing 36 is retained by use of the snap-fit projection 33c as described later. The snap-fit projection 33c extends in a circumferential direction, and is disposed close to a distal end of the end shell 22.

The fluid nozzle unit 26 includes the nozzle housing 36, a nozzle spout device 37 and an annular sealing device 38. The nozzle housing 36 is fitted in the holder cavity 33. The nozzle spout device 37 is fixedly secured to the nozzle housing 36. The annular sealing device 38 is disposed on a cylindrical peripheral surface 36a of the nozzle housing 36. The nozzle housing 36 is formed from a plastic material or other insulating material. An inner space is formed in the nozzle housing 36, including a retaining hole 39, an air branch conduit 40, a water branch conduit 41 and a converging channel 42 or converging passage in a Y shape. The retaining hole 39 receives the nozzle spout device 37 for retention. The branch conduits 40 and 41 are connectable respectively with the air channel 34 and the water channel 35. The converging channel 42 is a space where the branch conduits 40 and 41 extend to converge. The branch conduits 40 and 41 are positioned in a proximal end of the nozzle housing 36. The retaining hole 39 is one port of the converging channel 42 open at a distal end.

The peripheral surface 36a is fitted on the inner surface 33a of the holder cavity 33. The peripheral surface 36a includes a snap-fit groove 36b (peripheral groove) and a peripheral groove 36c for receiving the annular sealing device. Both of those extend in a circumferential direction. The snap-fit groove 36b receives the snap-fit projection 33c of the end shell 22 for positioning. The snap-fit projection 33c prevents the nozzle housing 36 from dropping out of the holder cavity 33.

The annular sealing device 38 is fixedly positioned in the peripheral groove 36c. The annular sealing device 38 extends in a circumferential direction of the peripheral surface 36a. An example of the annular sealing device 38 is an O-ring as an easily available element. When the nozzle housing 36 becomes fitted in the holder cavity 33, the annular sealing device 38 is squeezed and depressed between the peripheral groove 36c and an inner surface of the holder cavity 33, and becomes tightly fitted on the inner surface 33a. This is effective in closing a gap between the holder cavity 33 and the nozzle housing 36 hermetically, or in an air-tight and water-tight manner.

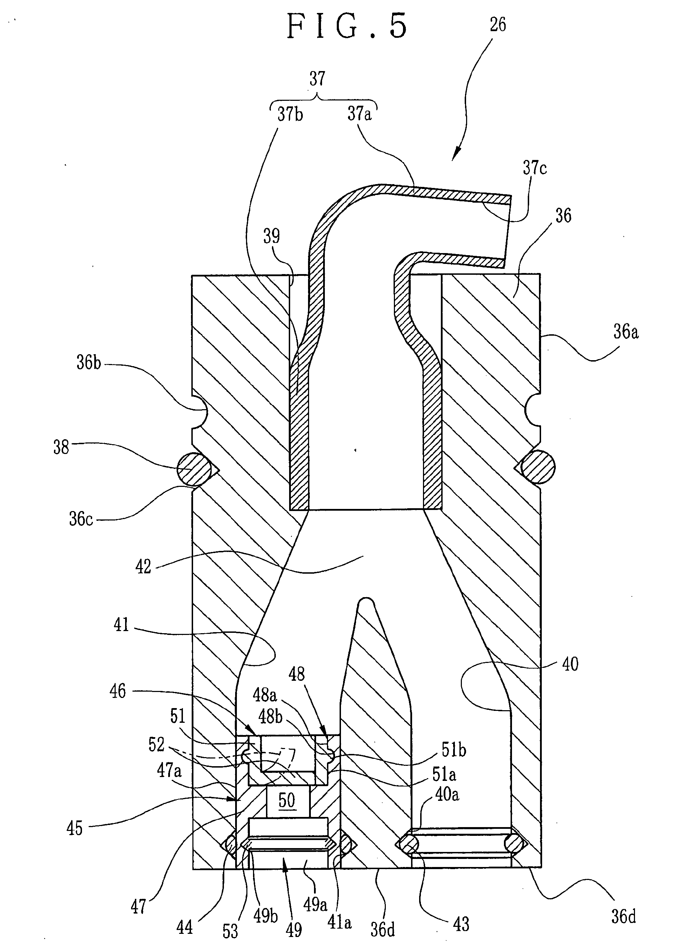

In Fig. 5, internal grooves 40a and 41a are formed in respectively the branch conduits 40 and 41, and extend in the circumferential direction. Annular sealing devices 43 and 44 are fitted in respectively the internal grooves 40a and 41a in a fixed manner. An inner diameter of the air branch conduit 40 is determined according to an outer diameter of the air flow sleeve 34a of the air channel 34.

A check valve device 45 is contained in the water branch conduit 41, and includes a valve sleeve 46 and valve housing 47. The valve housing 47 is cylindrical, and supports the valve sleeve 46. A peripheral surface 47a of the valve housing 47 is fitted on an inner surface of the water branch conduit 41 for fixation at its proximal end. The annular sealing device 44 is depressed forcibly when the valve housing 47 is fitted in the water branch conduit 41, for tight contact on the peripheral surface 47a of the valve housing 47. Thus, a gap between the water branch conduit 41 and the valve housing 47 can be closed in an air-tight and water-tight manner.

The valve housing 47 has a valve opening 50, a housing sleeve 48, and a fluid port 49 for connection. The valve opening 50 constitutes a flow chamber in the valve housing 47. The housing sleeve 48 is disposed at a distal end of the valve housing 47, and supports the valve sleeve 46. The housing sleeve 48 includes an inner surface 48a and a receiving groove 48b, which is formed in the inner surface 48a and extends in the circumferential direction.

The valve sleeve 46 is a piece of rubber or other elastic material, and includes a valve seat 51 (mount ring) and a valve membrane 52 (valve head) . The valve seat 51 is fitted in the inner surface 48a of the housing sleeve 48. The valve membrane 52 is disposed at a proximal end of the valve seat 51. The valve membrane 52 has a first end connected with the valve seat 51 and a second end free from the valve seat 51. The valve membrane 52, when in a normal state, is in a closed position inside the valve seat 51 to close the valve opening 50 by its resiliency as indicated by the solid line in Fig. 5, and upon receiving pressure of the cleaning water supplied by the water channel 35, comes away from the valve seat 51 to an open position of the phantom line in Fig. 5 to open the valve opening 50. The valve seat 51 (mount ring) includes a peripheral surface 51a and a retaining projection 51b formed on the peripheral surface 51a. The peripheral surface 51a is fitted on the inner surface 48a of the housing sleeve 48. The retaining projection 51b is retained in the receiving groove 48b to hold the valve sleeve 46 firmly in the housing sleeve 48.

The fluid port 49 is disposed at a proximal end of the valve housing 47, and connected with the water flow sleeve 35a of the water channel 35. The fluid port 49 has an inner surface 49a, on which an annular sealing device 53 is fitted. An inner diameter of the inner surface 49a is determined according to an outer diameter of the water channel 35. An internal groove 49b is formed in the inner surface 49a. The annular sealing device 53 is received in the internal groove 49b and retained firmly.

The nozzle housing 36 has a proximal surface 36d. When the fluid nozzle unit 26 is entered for contact of the proximal surface 36d with the base surface 33b of the holder cavity 33, the air flow sleeve 34a becomes coupled with the air branch conduit 40. The water flow sleeve 35a becomes coupled with the fluid port 49 of the check valve device 45. Also, the annular sealing device 43 tightly contacts the air flow sleeve 34a. The annular sealing device 53 tightly contacts the water flow sleeve 35a. Therefore, the fluid nozzle unit 26 can be connected to the air channel 34 and the water channel 35 hermetically, or in an air-tight and water-tight manner.

The nozzle spout device 37 is directed to eject water and air toward the imaging window area 24. The nozzle spout device 37 is formed from metal or other rigid material, and includes a spout opening 37c, a spout sleeve 37a and a port sleeve 37b. The port sleeve 37b is received in the retaining hole 39 of the nozzle housing 36, and communicates with a port of the converging channel 42. The spout sleeve 37a extends from the port sleeve 37b with a bend toward the spout opening 37c, and is positioned through the end opening 23d in the head cap 23 to appear externally.

Air from the air channel 34 is drawn to the nozzle spout device 37 after passing the air branch conduit 40 and the converging channel 42, and blows the imaging window area 24. Water from the water channel 35 is drawn to the nozzle spout device 37 after passing the valve sleeve 46, the water branch conduit 41 and the converging channel 42, and blows the imaging window area 24. When no water is supplied from the water channel 35, the valve membrane 52 is in a closed state and prevents backflow of fluid with dirt into the water channel 35. It is possible to remove dirt from the imaging window area 24 by ejection of water and air from the nozzle spout device 37 to the imaging window area 24.

After the imaging, the endoscope 10 is cleaned. If dirt deposits on the nozzle spout device 37, the fluid nozzle unit 26 is removed and abandoned before cleaning the endoscope 10. For the removal, the nozzle spout device 37 is picked up by use of a tool such as tweezers, to pull up the fluid nozzle unit 26 in the axial direction. The peripheral surface 36a of the nozzle housing 36 slides in the axial direction while pressed by the snap-fit projection 33c, to separate the fluid nozzle unit 26 from the holder cavity 33. The endoscope 10 after removing the fluid nozzle unit 26 is cleaned by a washing machine. After cleaning, a new fluid nozzle unit 26 is mounted in the holder cavity 33. To this end, the proximal surface 36d of the nozzle housing 36 is entered to reach the base surface 33b of the holder cavity 33. The air flow sleeve 34a is coupled to the air branch conduit 40. The water flow sleeve 35a is coupled to the fluid port 49 of the check valve device 45.

Therefore, it is easy to clean up the endoscope 10 because the fluid nozzle unit 26 can be removed and abandoned if the nozzle spout device 37 is clogged (blocked) by dirt or the like. As the converging channel 42 is formed in the fluid nozzle unit 26, the portion of the converging channel 42 which is difficult to clean up is abandoned together with the fluid nozzle unit 26. The cleanup can be performed efficiently even in the presence of dirt deposited within the nozzle spout device 37. Also, the air channel 34 and the water channel 35 become uncovered when the fluid nozzle unit 26 is removed from the holder cavity 33. It is possible to clean the air channel 34 and the water channel 35 even in the presence of dirt therein. The air channel 34 and the water channel 35 of the endoscope 10 can be brushed for cleaning, because the fluid nozzle unit 26 which cannot be brushed easily is removed from the endoscope 10.

In the above embodiment, the snap-fit projection 33c of the annular shape is used. Instead of this, pins can be formed to project from the inner surface 33a of the holder cavity 33, and used for positioning by engagement with the snap-fit groove 36b. Furthermore, plungers with springs can be used for the same purpose.

In the above embodiment, the nozzle housing 36 has the snap-fit groove 36b. The holder cavity 33 has the snap-fit projection 33c. Alternatively, a snap-fit projection can be formed on the peripheral surface 36a of the nozzle housing 36. A snap-fit groove can be formed in the inner surface 33a of the holder cavity 33, and engaged with the snap-fit projection.

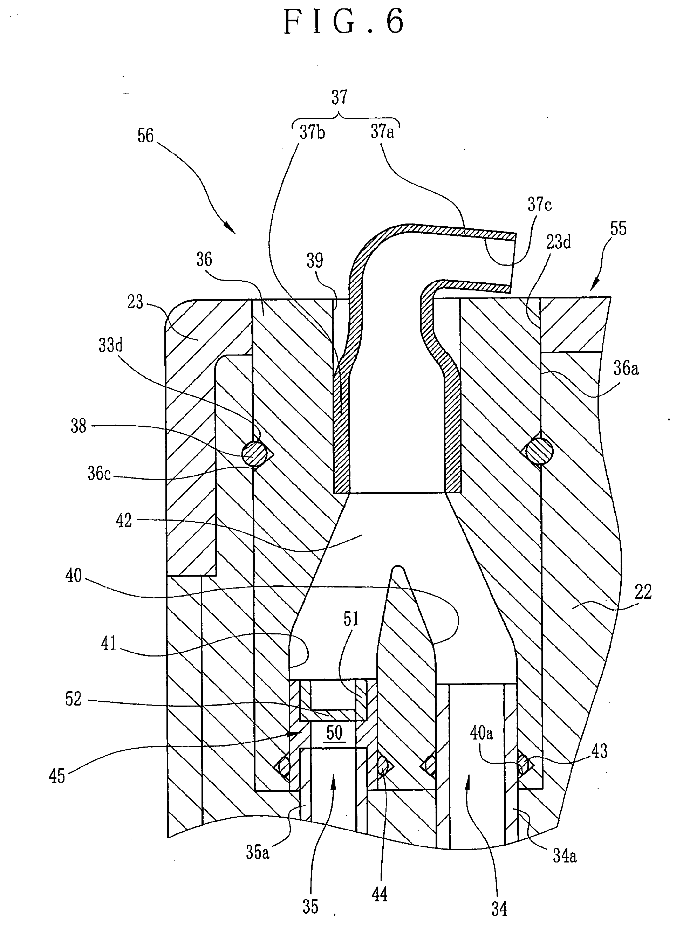

In the above embodiment, the snap-fit groove 36b (peripheral groove) operates for keeping the nozzle housing 36 in the holder cavity 33. In Fig. 6, another preferred fluid nozzle unit 56 of a cartridge type for a head assembly 55 is illustrated. The annular sealing device 38 disposed on the peripheral surface 36a of the nozzle housing 36 operates for positioning. A snap-fit groove 33d (internal groove) is formed in the inner surface 33a of the holder cavity 33 for firmly positioning the annular sealing device 38. This is also effective in keeping the nozzle housing 36 in the holder cavity 33 in a manner similar to the above embodiment.

In the above embodiment, the nozzle housing 36 and the holder cavity 33 are cylindrical. However, the nozzle housing 36 and the holder cavity 33 can be formed in other shapes, such as a shape of a polygonal prism.

In the above embodiments, the nozzle spout device 37 is originally separate from the nozzle housing 36 and attached to the nozzle housing 36 for a single unit. However, the nozzle spout device 37 can be a portion included in the piece of the nozzle housing 36.

Furthermore, it is possible to construct the nozzle spout device 37 in a form removable from the nozzle housing 36. This is effective in facilitating abandonment or reuse of any one of the nozzle housing 36 and the nozzle spout device 37.

In the above embodiments, the check valve device 45 is contained in the water branch conduit 41. However, the check valve device 45 can be incorporated in the air branch conduit 40. Also, two check valve devices 45 can be used and incorporated in respectively the branch conduits 40 and 41.

In the above embodiments, air and water are ejected by the fluid nozzle unit. However, other fluid can be ejected for cleaning the imaging window area, such as physiological saline solution, or fluid containing water and cleaning agent mixed in the water. In the above embodiments, the endoscope includes the image sensor for imaging. However, an endoscope of the invention can be a type in which an optical image guide device is used for imaging an object.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A fluid nozzle unit for an endoscope (10) including a section of an elongated tube (14) for entry in a body cavity, an end shell (22) disposed on a distal side of said elongated tube, and having a distal surface (23a), an imaging window means (24), provided in said distal surface, for endoscopic imaging in said body cavity, a liquid channel (35), formed through said elongated tube, for supplying liquid toward said distal surface, a gas channel (34), formed through said elongated tube, for supplying gas toward said distal surface, said fluid nozzle unit comprising:
a nozzle housing (36) for mounting on said end shell in a removable manner;
a liquid branch conduit (41), formed in said nozzle housing, and supplied with said liquid by said liquid channel;
a gas branch conduit (40), formed in said nozzle housing, and supplied with said gas by said gas channel; and
a converging channel (42), formed in said nozzle housing, for coupling said liquid branch conduit and said gas branch conduit together;
**characterized by**
a nozzle spout device (37), secured to said nozzle housing, disposed on said distal surface, for ejecting said liquid and said gas from said converging channel toward said imaging window means, and
a check valve device (45), incorporated in said liquid branch conduit (41), for preventing backflow of liquid.

2. A fluid nozzle unit as defined in claim 1, wherein said endoscope includes a holder cavity formed in said end shell to open in said distal surface;
wherein said nozzle housing is contained in said holder cavity.

3. A fluid nozzle unit as defined in claim 1, further comprising a retaining hole formed in said nozzle housing to communicate from said converging channel toward said distal surface;
wherein said nozzle spout device includes:
a port sleeve, retained in said retaining hole, and connected with said converging channel;
a spout sleeve disposed on said distal surface to extend from a distal end of said port sleeve toward said imaging window means.

4. A fluid nozzle unit as defined in claim 2, further comprising a positioning device for positioning said nozzle housing in said holder cavity to prevent drop therefrom.

5. A fluid nozzle unit as defined in claim 2, further comprising a sealing device disposed on a peripheral surface of said nozzle housing, for hermetically closing a gap between said nozzle housing and an inner surface of said holder cavity.

6. A fluid nozzle unit as defined in claim 5, further comprising a peripheral groove, formed in said peripheral surface, for receiving said sealing device;
wherein said endoscope includes an internal groove, formed in said inner surface of said holder cavity, opposed to said peripheral groove, for receiving said sealing device so as to position said nozzle housing in said holder cavity.

7. A fluid nozzle unit as defined in claim 1, wherein said check valve device (45) includes:
a valve opening for passing said liquid from said liquid channel;
a valve seat disposed at said valve opening;
a valve membrane, secured to said valve opening, shiftable from a closed position to an open position upon being pressed, for engagement with said valve seat when in said closed position, to close said valve opening and prevent passage of liquid from said converging channel, and for coming away from said valve seat when in said open position, to open said valve opening.

8. A fluid nozzle unit as defined in claim 2, wherein said endoscope includes:
a head cap, secured to said end shell, and positioned on said distal surface;
a first end opening, formed in said head cap, for setting said imaging window means to appear externally;
a second end opening formed in said head cap to correspond to said holder cavity.

9. An endoscope comprising the fluid nozzle unit as defined in any of claims 1 to 8.

## Patentansprüche

1. Fluiddüseneinheit für ein Endoskop (10), das einen Abschnitt eines länglichen Schlauchs (14) für den Eintritt in einen Körperhohlraum aufweist, ferner eine Endhülse (22), die sich auf einer distalen Seite des länglichen Schlauchs befindet und die eine distale Oberfläche (23a) aufweist, eine Abbildungsfenstereinrichtung (24), die in der distalen Oberfläche vorgesehen ist für eine endoskopische Bildgebung innerhalb des Körperhohlraums, einen Flüssigkeitskanal (35), der durch den länglichen Schlauch hindurch ausgebildet ist, um Flüssigkeit zu der distalen Oberfläche zu liefern und einen Gaskanal (34), der durch den länglichen Schlauch hindurch ausgebildet ist, um Gas in Richtung der distalen Oberfläche zu liefern, wobei die Fluiddüseneinheit umfasst:
ein Düsengehäuse (36) zum abnehmbaren Halten an der Endhülse;
eine Flüssigkeitsverzweigungsleitung (41), die in dem Düsengehäuse ausgebildet ist und von dem Flüssigkeitskanal mit Flüssigkeit gespeist wird;
eine Gasverzweigungsleitung (40), die in dem Düsengehäuse ausgebildet ist und von dem Gaskanal mit Gas gespeist wird; und
einen in dem Düsengehäuse ausgebildeten Zusammenflusskanal (42) zum Koppeln der Flüssigkeitsverteilungsleitung und der Gasverzweigungsleitung;
**gekennzeichnet durch**
eine Düsenmündungseinrichtung (37), die an dem Düsengehäuse befestigt und an der distalen Oberfläche gelegen ist, um die Flüssigkeit und das Gas aus dem Zusammenflusskanal in Richtung der Abbildungsfenstereinrichtung auszustoßen, und
eine Rückschlagventileinrichtung (45), die in die Flüssigkeitsverzweigungsleitung (41) eingefügt ist, um einen Rückstrom der Flüssigkeit zu unterbinden.

2. Fluiddüseneinheit nach Anspruch 1, bei der das Endoskop einen Aufnahmehohlraum enthält, der in der Endhülse ausgebildet ist, um in die distale Oberfläche zu münden;
wobei das Düsengehäuse in dem Aufnahmehohlraum enthalten ist.

3. Fluiddüseneinheit nach Anspruch 1, weiterhin umfassend ein in dem Düsengehäuse ausgebildetes Rückhalteloch für die Verbindung von dem Zusammenflusskanal in Richtung auf die distale Oberfläche;
wobei die Düsenmündungseinrichtung enthält:
eine in dem Rückhalteloch gehaltene Öffnungshülse, die mit dem Zusammenflusskanal verbunden ist;
eine Mündungshülse, die in der distalen Oberfläche angeordnet ist, um sich von einem distalen Ende der Öffnungshülse in Richtung der Abbildungsfenstereinrichtung zu erstrecken.

4. Fluiddüseneinheit nach Anspruch 2, weiterhin umfassend eine Positioniereinrichtung zum Positionieren des Düsengehäuses in dem Aufnahmehohlraum, um ein Herausfallen aus diesem zu verhindern.

5. Fluiddüseneinheit nach Anspruch 2, weiterhin umfassend eine Dichtungseinrichtung, die an einer Umfangsfläche des Düsengehäuses angeordnet ist, um eine Lücke zwischen dem Düsengehäuse und einer Innenoberfläche des Aufnahmehohlraums hermetisch zu verschließen.

6. Fluiddüseneinheit nach Anspruch 5, weiterhin umfassend eine Umfangsnut, die in der Umfangsfläche ausgebildet ist, um die Dichtungseinrichtung aufzunehmen;
wobei das Endoskop eine Innennut enthält, die in der Innenfläche des Aufnahmehohlraums gegenüber der Umfangsnut ausgebildet ist, um die Dichtungseinrichtung aufzunehmen und dadurch das Düsengehäuse in dem Haltehohlraum zu positionieren.

7. Fluiddüseneinheit nach Anspruch 1, bei der die Rückschlagventileinrichtung (45) aufweist:
eine Ventilöffnung zum Durchlassen der Flüssigkeit aus dem Flüssigkeitskanal;
einen in der Ventilöffnung angeordneten Ventilsitz;
eine an der Ventilöffnung befestigte Ventilmembran, verschiebbar aus einer geschlossenen Stellung in eine geöffnete Stellung bei Druckbeaufschlagung, um in der geschlossenen Stellung mit dem Ventilsitz zusammenzuwirken und die Ventilöffnung zu schließen und so den Durchgang von Flüssigkeit aus dem Zusammenflusskanal zu unterbinden, und um in der geöffneten Stellung von dem Ventilsitz abzurücken, um die Ventilöffnung zu öffnen.

8. Fluiddüseneinheit nach Anspruch 2, bei der das Endoskop enthält:
eine an der Endhülse befestigte Kopfkappe, die an der distalen Oberfläche gelegen ist;
eine erste Endöffnung, die in der Kopfkappe ausgebildet ist, um die Abbildungsfenstereinrichtung nach außen in Erscheinung treten zu lassen;
eine zweite Endöffnung, die in der Kopfkappe ausgebildet ist, um dem Haltehohlraum zu entsprechen.

9. Endoskop mit einer Fluiddüseneinheit nach einem der Ansprüche 1 bis 8.

## Revendications

1. Unité de buse de fluide pour endoscope (10) comprenant une section d'un tube allongé (14) pour l'entrée dans une cavité corporelle, une coque d'extrémité (22) disposée sur un côté distal dudit tube allongé, et ayant une surface distale (23a), des moyens de fenêtre d'imagerie (24) prévus dans ladite surface distale, pour l'imagerie endoscopique dans ladite cavité corporelle, un canal de liquide (35) formé à travers ledit tube allongé, pour amener le liquide vers ladite surface distale, un canal de gaz (34) formé à travers ledit tube allongé, pour amener le gaz vers ladite surface distale, ladite unité de buse de fluide comprenant :
un boîtier de buse (36) destiné à être monté sur ladite coque d'extrémité d'une manière amovible ;
un conduit de bifurcation de liquide (41) formé dans ledit boîtier de buse, et alimenté avec ledit liquide par ledit canal de liquide ;
un conduit de bifurcation de gaz (40) formé dans ledit boîtier de buse, et alimenté avec ledit gaz par ledit canal de gaz ; et
un canal convergent (42) formé dans ledit boîtier de buse, pour coupler ledit conduit de bifurcation de liquide et ledit conduit de bifurcation de gaz ensemble ;
**caractérisée par** :
un dispositif formant bec verseur de buse (37) fixé sur ledit boîtier de buse, disposé sur ladite surface distale, pour éjecter ledit liquide et ledit gaz dudit canal convergent vers lesdits moyens de fenêtre d'imagerie, et
un dispositif formant clapet anti-retour (45) incorporé dans ledit conduit de bifurcation de liquide (41) pour empêcher le refoulement du liquide.

2. Unité de buse de fluide selon la revendication 1, dans laquelle ledit endoscope comprend une cavité de support formée dans ladite coque d'extrémité pour s'ouvrir dans ladite surface distale ;
dans laquelle ledit boîtier de buse est contenu dans ladite cavité de support.

3. Unité de buse de fluide selon la revendication 1, comprenant en outre un trou de retenue formé dans ledit boîtier de buse pour communiquer dudit canal convergent vers ladite surface distale ;
dans laquelle ledit dispositif formant bec verseur de buse comprend :
un manchon d'orifice, retenu dans ledit trou de retenue, et raccordé avec ledit canal convergent ;
un manchon de bec verseur disposé sur ladite surface distale pour s'étendre d'une extrémité distale dudit manchon d'orifice vers lesdits moyens de fenêtre d'imagerie.

4. Unité de buse de fluide selon la revendication 2, comprenant en outre un dispositif de positionnement pour positionner ledit boîtier de buse dans ladite cavité de support pour empêcher sa chute.

5. Unité de buse de fluide selon la revendication 2, comprenant en outre un dispositif d'étanchéité disposé sur une surface périphérique dudit boîtier de buse, afin de fermer hermétiquement un espace entre ledit boîtier de buse et une surface interne de ladite cavité de support.

6. Unité de buse de fluide selon la revendication 5, comprenant en outre une rainure périphérique, formée dans ladite surface périphérique, pour recevoir ledit dispositif d'étanchéité ;
dans laquelle ledit endoscope comprend une rainure interne, formée dans ladite surface interne de ladite cavité de support, opposée à ladite rainure périphérique, pour recevoir ledit dispositif d'étanchéité afin de positionner ledit boîtier de buse dans ladite cavité de support.

7. Unité de buse de fluide selon la revendication 1, dans laquelle ledit dispositif formant clapet anti-retour (45) comprend :
une ouverture de clapet pour laisser passer ledit liquide dudit canal de liquide ;
un siège de clapet disposé au niveau de ladite ouverture de clapet ;
une membrane de clapet, fixée sur ladite ouverture de clapet, pouvant passer d'une position fermée à une position ouverte après avoir été enfoncée, pour la mise en prise avec ledit siège de clapet lorsqu'il est dans ladite position fermée, afin de fermer ladite ouverture de clapet et empêcher le passage de liquide dudit canal convergent ou pour se détacher dudit siège de clapet, lorsqu'il est dans ladite position ouverte, afin d'ouvrir ladite ouverture de clapet.

8. Unité de buse de fluide selon la revendication 2, dans laquelle ledit endoscope comprend :
un capuchon de tête, fixé sur ladite coque d'extrémité, et positionné sur ladite surface distale ;
une première ouverture d'extrémité, formée dans ledit capuchon de tête, pour placer lesdits moyens de fenêtre d'imagerie afin qu'ils apparaissent à l'extérieur ;
une seconde ouverture d'extrémité formée dans ledit capuchon de tête pour correspondre à ladite cavité de support.

9. Endoscope comprenant l'unité de buse de fluide selon l'une quelconque des revendications 1 à 8.
